# EUROPEAN PATENT APPLICATION

(11) **EP 4 846 963 A2**
(43) Date of publication of application: **02.12.2026**
(21) Application number: 26212510.7
(22) Date of filing: 22.11.2022
(51) Int. Cl.: A61B 18/02

(54) **CAPACITY MEASURING DEVICES FOR CRYOGEN VESSELS, AND RELATED SYSTEMS**

(30) Priority: 24.11.2021 US 202163282903 P
(62) Document number(s) of the earlier application(s) in accordance with Art. 76 EPC: 22839098.5 / 4 436 504
(71) Applicant: Hologic, Inc., Marlborough, MA 01752 (US)
(72) Inventor: DEFREITAS, Kenneth, Marlborough, Massachusetts, 01752 (US); GKANATSIOS, Nikolaos, Marlborough, Massachusetts, 01752 (US)
(74) Representative: Abel & Imray LLP

(57) **Abstract**

A cryogen delivery system includes a probe configured to deliver a cryogenic fluid to a treatment location, a cryogen inlet configured to interface with a cryogenic fluid supply vessel to transfer cryogenic fluid from the cryogenic fluid supply vessel to the probe, a support structure configured to position the cryogenic fluid supply vessel relative to the cryogen inlet, and a sensor coupled to the support structure. The sensor is configured to generate a signal indicative of a weight of a cryogenic fluid contained in the cryogenic fluid supply vessel. A control system operably coupled to the sensor is configured to sense an amount of cryogenic fluid in the cryogenic fluid supply vessel only when the cryogenic fluid supply vessel is not interfaced with the cryogen inlet.

## Description

### Technical Field

The present disclosure generally relates to the field of cryoablation devices and systems. More specifically, the disclosure relates to devices and methods for determining an amount of cryogen contained in a cryogenic fluid supply vessel for use during a cryoablation procedure.

### Introduction

The section headings used herein are for organizational purposes only and are not to be construed as limiting the subject matter described in any way.

Cryogen delivery systems, including systems with cryosurgical probes, are used to destroy diseased or abnormal tissue cells to treat a variety of medical conditions. When cryogenic liquids such as liquid nitrogen are used with a cryosurgical probe, tissues adjacent to the probe quickly freeze, causing the tissue to die, after which it is absorbed by the body, expelled by the body, or sloughed off. Cryogenic delivery systems can be used to treat, among other things, skin cancer, skin lesions, breast tumors (both benign and cancerous), prostate cancer and benign prostate disease, liver tumors and liver cancer, glaucoma and other eye diseases, and other conditions.

Cryogen delivery systems such as cryoablation devices receive a supply of a cryogen, such as liquid nitrogen or other cryogenic fluids, from a fluid supply vessel. The cryogen is consumed during a cryogenic procedure and, once the fluid supply vessel is empty, further operation of the delivery system requires the empty fluid supply vessel to be replaced with another vessel containing cryogenic fluid. Some cryoablation procedures include an alternating cycle of freezing and thawing intervals, such as two freezing cycles with a passive thawing interval in between the freezing cycles. If the cryogen is consumed prior to the desired end time of the procedure, the tissue mass desired to be frozen and destroyed may not be sufficiently cooled, and the procedure may not be completed successfully or to the degree desired or expected.

There is a need for devices and methods for providing information to the operator of a cryogen delivery system regarding the amount of cryogen available for use for a procedure. There is a further need to provide this information to the operator in a manner in which the operator can determine whether the available amount of cryogen is sufficient to complete or initiate a planned procedure.

### Summary

The present disclosure addresses one or more of the above-mentioned problems and/or achieves one or more of the above-mentioned desirable features. Other features and/or advantages may become apparent from the description which follows.

In accordance with various exemplary embodiments of the present disclosure, a cryogen delivery system includes a probe configured to deliver a cryogenic fluid to a treatment location, a cryogen inlet configured to interface with a cryogenic fluid supply vessel to transfer cryogenic fluid from the cryogenic fluid supply vessel to the probe, a support structure configured to position the cryogenic fluid supply vessel relative to the cryogen inlet, and a sensor coupled to the support structure. The sensor is configured to generate a signal indicative of a weight of a cryogenic fluid contained in the cryogenic fluid supply vessel. In an example, the sensor comprises a strain gauge sensor.

In another example, the support structure is a moveable support structure configured to move the cryogenic fluid supply vessel from a first position to a second position, the second position being a position in which the cryogenic fluid supply vessel is interfaced with the cryogen inlet. The moveable support structure comprises, for example, a pneumatic lift or an electromechanical lift. In still another example, the cryogen delivery system further comprises a visual interface configured to display information based on the signal indicative of the weight of the cryogenic fluid contained in the cryogenic fluid supply vessel. The visual interface is, for example, configured to display a volume of the cryogenic fluid present in the cryogenic fluid supply vessel based on the signal indicative of the weight of the cryogenic fluid contained in the cryogenic fluid supply vessel. In yet another example, the visual interface is configured to display an amount of run time for operation of the cryogen delivery system based on the signal indicative of the weight of the cryogenic fluid contained in the cryogenic fluid supply vessel. In an additional example, the visual interface is configured to display a percentage representative of an amount of cryogenic fluid present in the cryogenic fluid supply vessel, based on the signal indicative of the weight of the cryogenic fluid, as compared to an amount of cryogenic fluid present in a full cryogenic fluid supply vessel.

In another example, the cryogen delivery system is configured to perform cryoablation. The cryogen delivery system is, for example, configured to perform a plurality of cooling cycles in which the probe delivers the cryogenic fluid to the treatment location. In still another example, the cryogen delivery system is configured to compare an amount of cryogenic fluid present in the cryogenic fluid supply vessel with an amount of cryogenic fluid available to perform a procedure. In yet another example, the cryogen delivery system is configured to indicate whether the amount of cryogenic fluid present in the cryogenic fluid supply vessel is sufficient to complete a selected procedure.

In an additional example, the cryogenic fluid supply vessel comprises a plurality of cryogenic fluid supply vessels; and the cryogen delivery system comprises an automated fluid supply vessel switching system configured to move a first cryogenic fluid supply vessel of the plurality of cryogenic fluid supply vessels from the support structure and place a second cryogenic fluid supply vessel of the plurality of cryogenic fluid supply vessels on the support structure based at least in part on a weight of a cryogenic fluid contained in the first cryogenic fluid supply vessel.

In accordance with another exemplary embodiment of the present disclosure, a method for determining an amount of cryogenic fluid in a cryogenic fluid supply vessel comprises generating a signal from a sensor coupled to a support structure on which a cryogenic fluid supply vessel is located, the signal being indicative of a total weight of the cryogenic fluid supply vessel, and determining an amount of cryogenic fluid present in the cryogenic fluid supply vessel based on the signal. In an example, generating a signal from a sensor coupled to the support structure comprises generating a signal from a strain gauge sensor coupled to the support structure. In another example, determining an amount of a cryogenic fluid in the cryogenic fluid supply vessel comprises subtracting an estimated weight of an empty cryogenic fluid supply vessel from the total weight of the cryogenic fluid supply vessel. In still another example, the method further comprises providing an indication regarding the amount of cryogenic fluid present in the cryogenic fluid supply vessel. In yet another example, providing an indication regarding the amount of cryogenic fluid present in the cryogenic fluid supply vessel comprises one or more of a visible indication and an audible indication. In an additional example, providing an indication regarding the amount of cryogenic fluid present in the cryogenic fluid supply vessel includes displaying the indication on a display associated with the cryogen delivery system.

In another example, providing the indication regarding the amount of cryogenic fluid present in the cryogenic fluid supply vessel comprises providing an indication of the amount of cryogenic fluid present in the cryogenic fluid supply vessel by weight. In still another example, providing the indication regarding the amount of cryogenic fluid present in the cryogenic fluid supply vessel comprises providing an indication of the amount of cryogenic fluid present in the cryogenic fluid supply vessel by volume. In yet another example, providing the indication regarding the amount of cryogenic fluid present in the cryogenic fluid supply vessel comprises providing an indication the amount of cryogenic fluid present is sufficient to complete a freezing cycle of the cryogen delivery system, providing an indication the amount of cryogenic fluid present is insufficient to complete a freezing cycle of the cryogen delivery system; or providing an indication that the amount of cryogenic fluid present is sufficient to complete two freezing cycles of the cryogen delivery system.

In accordance with yet another exemplary embodiment of the present disclosure, a method for determining sufficiency of an amount of cryogen for performing a procedure with a cryogen delivery system comprises determining a weight of a cryogenic fluid contained in a cryogenic fluid supply vessel of the cryogen delivery system based on a signal from a sensor, determining an amount of time the cryogen delivery system can operate based on the weight of the cryogenic fluid, and providing, via an indicator of the cryogen delivery system, an indication of the amount of time the cryogen delivery system can operate. In an example, determining a weight of a cryogenic fluid contained in a cryogenic fluid supply vessel comprises determining a total weight of the cryogenic fluid supply vessel and the cryogenic fluid; and subtracting the weight of the cryogenic fluid supply vessel from the total weight. In another example, providing an indication of the amount of time the cryogen delivery system can operate comprises displaying a visual indicator on a display operatively associated with the cryogen delivery system.

In still another example, the method further comprises receiving an input at a user input device of the cryogen delivery system indicative of a selected cryoablation procedure; determining a runtime of the selected cryoablation procedure; and determining whether the amount of time the cryogen delivery system can operate is less than, equal to, or greater than the runtime of the selected cryoablation procedure. In yet another example, on the condition the amount of time the cryogen delivery system can operate is less than the runtime of the selected cryoablation procedure, providing an indication of the amount of time the cryogen delivery system can operate comprises providing an indication that the amount of cryogen is insufficient to perform the selected cryoablation procedure. In yet another example, on the condition the amount of time the cryogen delivery system can operate is greater than the runtime of the selected cryoablation procedure, providing an indication of the amount of time the cryogen delivery system can operate comprises providing an indication that the amount of cryogen is sufficient to perform the selected cryoablation procedure. In an additional example, the selected cryoablation procedure includes a first runtime corresponding to a first freezing cycle and a second runtime corresponding to a second freezing cycle and, wherein, determining whether the amount of time the cryogen delivery system can operate is less than, equal to, or greater than the runtime of the selected cryoablation procedure comprises determining whether the amount of time the cryogen delivery system can operate is less than, equal to, or greater than the first runtime and/or the first and second runtimes.

Additional objects and advantages will be set forth in part in the description which follows, and in part will be obvious from the description, or may be learned by practice of the present teachings. At least some of the objects and advantages of the present disclosure may be realized and attained by means of the elements and combinations particularly pointed out in the appended claims.

Both the foregoing general description and the following detailed description are exemplary and explanatory only and are not restrictive of the present disclosure and claims, including equivalents. The present disclosure and claims, in their broadest sense, could be practiced without having one or more features of these exemplary aspects and embodiments.

### Brief Description of the Drawings

The accompanying drawings, which are incorporated in and constitute a part of this specification, illustrate some exemplary embodiments of the present disclosure and together with the description, serve to explain certain principles. These drawings depict only typical embodiments of the disclosed inventions and are not therefore to be considered limiting of its scope. In the drawings:
FIG. 1 is a perspective view of an example embodiment of a cryogen delivery system console in accordance with the present disclosure.
FIGS. 2A and 2B are schematic side views of a cryogen delivery system with a moveable support mechanism in a lowered position (FIG. 2A) and a raised position (FIG. 2B).
FIG. 3 is a partial, cross-sectional side view of a cryogenic fluid supply vessel, support mechanism, and sensor according to an embodiment of the present disclosure.
FIG. 4 illustrates a visual interface of a cryogenic delivery system according to an exemplary embodiment of the present disclosure.
FIG. 5 is a flow chart showing a workflow for a cryosurgical procedure according to an exemplary embodiment of the present disclosure.
FIG. 6 is a flow chart showing a workflow for a cryosurgical procedure according to another exemplary embodiment of the present disclosure.
FIG. 7 is a schematic view of an automated switching system for cryogenic fluid supply vessels according to an exemplary embodiment of the present disclosure.

### Detailed Description of the Illustrated Embodiments

Exemplary embodiments of the present disclosure include cryogen delivery systems configured for medical treatment or surgical use, such as cryosurgical or cryoablation systems. The cryogen delivery systems include features configured to provide information to an operator of the cryogen delivery system regarding an amount of cryogen present in a cryogenic fluid supply vessel, such as a dewar, flask, or other thermal container, prior to initiation of a cooling operation or procedure.

In some embodiments, the cryogen delivery system includes one or more sensors configured to generate a signal indicative of an overall weight of the vessel including the cryogen contained therein. The sensor can be coupled, for example, to a moveable support mechanism configured to raise and lower the cryogenic fluid supply vessel. The moveable support mechanism can be configured to create or break a seal between the cryogenic fluid supply vessel and a cryogen inlet portion of the cryogen delivery system.

The sensor can be operably coupled to a control system of the cryogen delivery system, and the information from the sensor can be processed and provided to the operator of the cryogen delivery system, e.g., via a visual interface such as a display, an audio indicator, or other indicator. The indicator can be text, color, icon, or a combination of all three on a display, and/or a warning light located on the console. Additionally, or alternatively, the indicator may include an audio component. The control system can be configured to provide the information from the sensor in terms of volume of cryogen contained in the vessel, weight of the cryogen contained in the vessel, a fractional indication of cryogen relative to a full container of cryogen, an estimated run time available, or other parameter. For example, in some embodiments, the cryogen delivery system can use the information indicative of the amount of cryogen in the supply vessel to determine, for example, a length of time the delivery system can run based on the amount of cryogen remaining in the vessel and a flow rate of the delivery system. Based on this determination, and dependent upon other input provided by an operator of the cryogen delivery system, the system can provide feedback to the system operator regarding a procedure to be initiated. The type of feedback may be general, such as an indication that sufficient cryogenic fluid is present, an indication that insufficient cryogenic fluid is present or instructions to connect a new (or different) cryogenic fluid supply vessel, or the feedback may be more specific, such as an indication of an amount of time the system can run given the amount of cryogenic fluid present, instructions to replace the cryogenic fluid supply vessel during a thawing phase of the procedure, or other feedback to guide the operator's use of the cryogenic delivery system.

In some example embodiments, the control system is configured to sense the amount of cryogen in the fluid supply vessel only when the vessel is not interfaced with the cryogenic fluid inlet. Such a configuration reduces the possibility of generating erroneous signals in the sensor based on reaction forces or forces acting against the vessel when the vessel is sealed against the cryogenic fluid inlet, e.g., responsive to force applied by the moveable support mechanism during connection of the cryogenic fluid supply vessel to the fluid inlet.

According to exemplary embodiments of the present disclosure, the sensor can be or can include a strain gauge sensor, a sensor including a plurality of strain gauges such as a load cell, a piezoelectric transducer, or other sensor. The sensor can be operably coupled to the control system, which can be or include one or more processors operably coupled with electronic storage elements such as random-access memory, read-only memory, or other storage mediums, and programmed with software to control the various functions of the cryogen delivery system.

In alternate example embodiments, one or more sensors may be used in a storage area of the cryogenic delivery system, in which the storage area is configured to support and store multiple cryogenic fluid supply vessels. Each portion of the storage area that is configured to support a cryogenic fluid supply vessel can have an individual sensor configured to generate a signal indicative of an overall weight of the vessel stored in that location. The sensors from each portion of the storage area can be operably coupled to a control system of the cryogen delivery system, and the information from the sensors can be processed and provided to the operator of the cryogen delivery system, e.g., via a visual interface such as a display, an audio indicator, or other indicator. In addition to the type of information indicated above, the control system may provide information to the operator of the system such as directing the loading/installation of a fluid supply vessel from a particular storage area based on the procedure parameters input by the operator.

Exemplary embodiments of the present disclosure can be used with, for example, cryogen delivery systems commercialized by Hologic, Inc., Marlborough, MA, USA, and described in connection with U.S. Patent No. 8,092,448, (filed April 27, 2007), the entire contents of which are incorporated by reference herein.

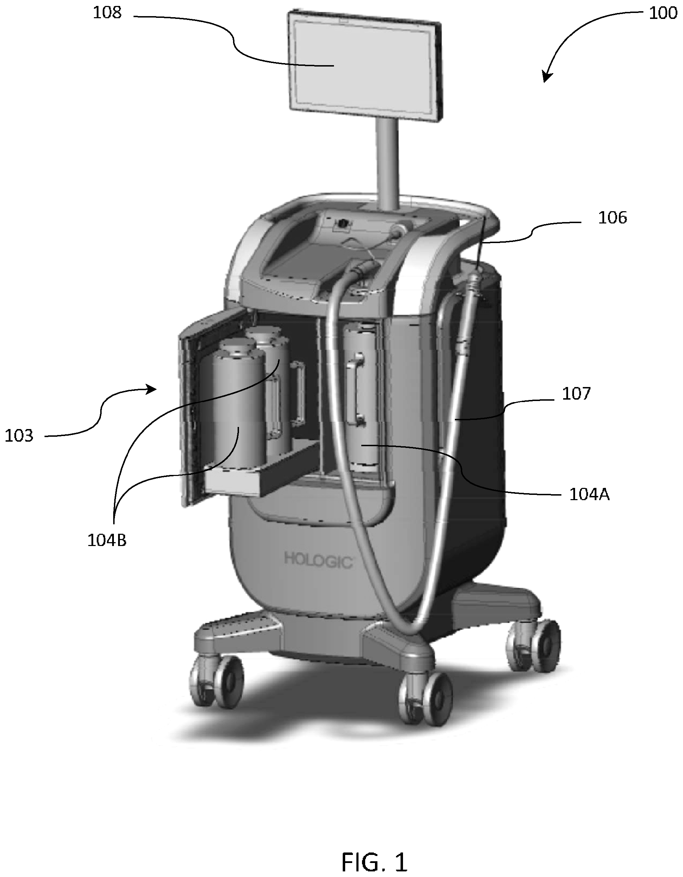

Referring now to FIG. 1, a cryogen delivery system 100 according to the present disclosure is shown. As shown in FIG. 1, the cryogen delivery system can be in the form of a wheeled console to facilitate ease of use and portability. In some embodiments, the cryogen delivery system 100 can be configured to perform, for example, a cryosurgical procedure such as cryoablation. The cryogen delivery system comprises a probe 106, a cryogen supply hose 107 to which the probe 106 is coupled, and a cryogenic fluid supply vessel 104A. The cryogen supply hose 107 is configured to receive a flow of cryogenic fluid from the cryogenic fluid supply vessel 104A for delivery through the probe 106 to a treatment location. The cryogen delivery system 100 includes a storage area 103 for storage of additional cryogenic fluid supply vessels 104B. The cryogen delivery system 100 also includes a visual interface, such as a display 108, configured to communicate information to the operator of the cryogen delivery system 100 regarding the operational state of the cryogen delivery system 100. In some embodiments, the display 108 may comprise touchscreen controls to enable the operator to alter or input operational parameters and otherwise control the cryogen delivery system 100.

According to various embodiments of the present disclosure, the display 108 may also be configured to display information regarding an amount of cryogen present or available in the cryogenic fluid supply vessel104A. Such information can be provided as an available weight of cryogen, an available volume of cryogen, a fraction of a full volume or weight (i.e., where the fraction represents a percentage of a full or unused amount of cryogen), or other indication. Alternatively, instead of providing an amount that is available, the indication may be presented as an amount remaining in a fluid supply vessel, where the cryogenic fluid in fluid supply vessels used in prior procedures can be partially depleted. In some embodiments, the display may provide an expected available possible runtime based on the available amount of cryogen.

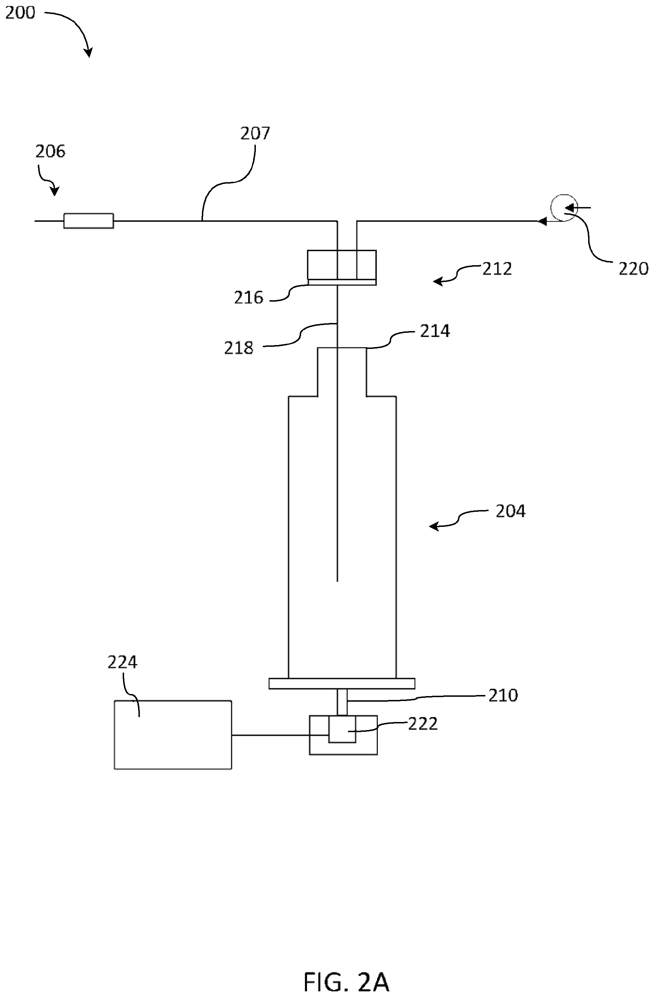

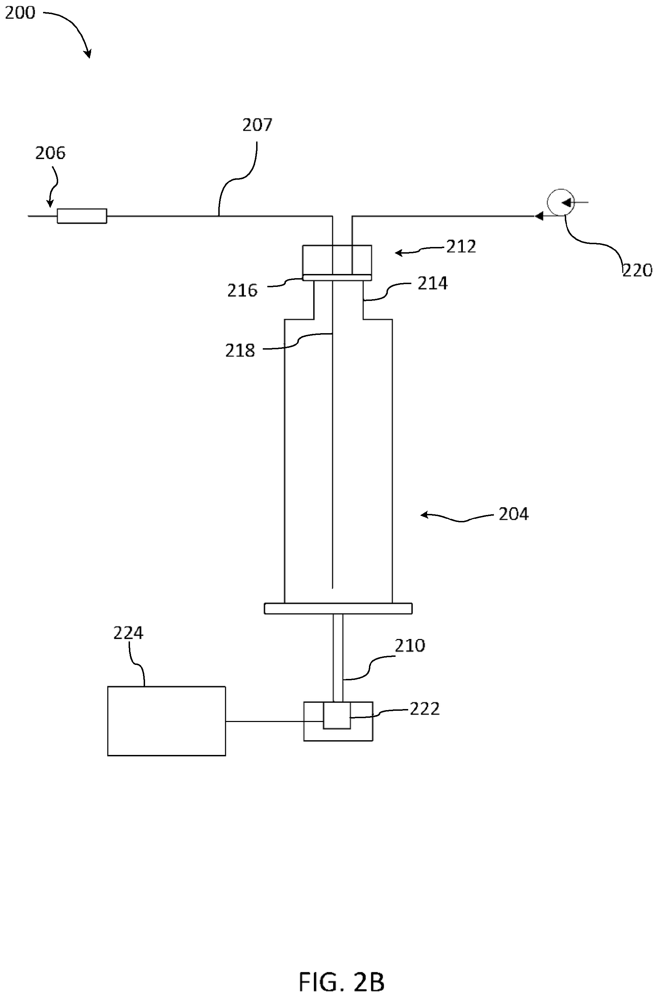

Referring now to FIG. 2A, a schematic view of a cryogen delivery system 200 is shown. A cryogenic fluid supply vessel 204 is positioned on a moveable support structure 210. The moveable support structure 210 is configured to raise and lower the cryogenic fluid supply vessel 204 relative to a cryogenic fluid inlet interface 212. The cryogenic fluid inlet interface 212 is configured to form a seal with a mouth 214 of the cryogenic fluid supply vessel 204 or otherwise configured to interface with the mouth 214 of the cryogenic fluid supply vessel 204. The moveable support structure 210 can be or include a platform or cup-shaped receptacle configured to receive and support the cryogenic fluid supply vessel 204 and coupled to a hydraulic lift, a pneumatic lift, an electromechanical actuator such as a motor-driven lead screw system, or other mechanism. In the lowered position shown in FIG. 2A, the cryogenic fluid supply vessel 204 can be placed on or removed from the moveable support structure 210 by an operator to prepare the cryogen delivery system 200 for use, e.g., by removing an empty or partially-used cryogenic fluid supply vessel from the moveable support structure 210 and replacing the empty or partially-used fluid supply vessel with a different fluid supply vessel, which may be a full or unused cryogenic fluid supply vessel. Once the cryogenic fluid supply vessel 204 is in place on the moveable support structure 210, the moveable support structure 210 can be actuated, e.g., by utilizing controls provided on the cryogen delivery system 200 (e.g., controls on display 108 shown in FIG. 1) to raise the cryogenic fluid supply vessel 204 from the position shown in FIG. 2A to the position shown in FIG. 2B. Alternatively, an operator may begin a procedure with a cryogenic fluid supply vessel in place from a prior use by the same or a different operator and may rely on the system to provide information regarding the amount of cryogen available as disclosed herein.

In the position shown in FIG. 2B, the mouth 214 of the cryogenic fluid supply vessel 204 forms a seal against the interface 212. In some embodiments, the interface 212 comprises a seal member 216 and a cryogen inlet tube 218. The seal member 216 may be configured to seal against the mouth 214 of the cryogenic fluid supply vessel 204 to prevent loss of the cryogen within the cryogenic fluid supply vessel 204, e.g., by evaporation. The cryogen inlet tube 218 can be a flexible tube or a rigid tube. The cryogen inlet tube 218 can be configured to extend to the bottom of the cryogenic fluid supply vessel 204 to ensure that the cryogen can be completely emptied or nearly completely emptied in use. In some embodiments, the cryogen inlet tube 218 is fixed in place in the interface 212, and the moveable support structure 210 can be configured with sufficient travel to lower to the cryogenic fluid supply vessel 204 far enough to clear the end of the cryogen inlet tube 218 at the lowered position. In other embodiments, the cryogen inlet tube 218 can be configured to be removed at least partially from the cryogenic fluid supply vessel 204, and the distance traveled by the moveable support structure 210 can be correspondingly less, e.g., only enough travel to clear whatever portion of the cryogen inlet tube 218 remains in the cryogenic fluid supply vessel 204 and remove the cryogenic fluid supply vessel 204 from the interface 212.

Once the cryogenic fluid supply vessel 204 is in the position shown in FIG. 2B, i.e., sealed with the interface 212, the interior of the cryogenic fluid supply vessel 204 can be pressurized (e.g., via an air pump 220 or another source of pressurized air or other gas) to force the cryogenic fluid from the cryogenic fluid supply vessel 204, through the cryogen inlet tube 218 and the cryogen supply hose 207, and out through the probe 206 to carry out the cryoablation procedure. As discussed in more detail in U.S. Patent 8,092,448, incorporated herein by reference above, a typical cryoablation procedure may include a series of alternating active freezing cycles, in which the cryogenic fluid is supplied to the probe 206 and the lesion or other tissue mass is frozen using the probe 206, and passive thawing cycles in which the supply of cryogenic fluid to probe 206 is paused. If the procedure is interrupted during a freezing cycle, or if a passive thawing cycle extends beyond an predetermined period of time, the overall success of the procedure may be negatively impacted, so it is desired to enable the operator to confirm, prior to beginning a procedure, that the cryogenic fluid supply vessel 204 contains enough cryogen for the entire procedure, or at least for one complete freezing cycle such that the cryogenic fluid supply vessel 204 can be exchanged during the passive thawing cycle.

Referring still to FIGS. 2A and 2B, according to embodiments of the present disclosure, the moveable support structure 210 can comprise a sensor 222 configured to generate a signal indicative of the overall weight of the cryogenic fluid supply vessel 204. The sensor 222 may be operably coupled to a control system 224 of the cryogen delivery system 200, and the control system 224 can be configured to receive the signal from the sensor 222 and process the signal to provide information to the operator of the cryogen delivery system 200 regarding the amount of cryogenic fluid present in the cryogenic fluid supply vessel 204. Such processing can include, for example, associating a voltage signal from the sensor 222 with a total overall weight of the cryogenic fluid supply vessel 204. Further processing can include, for example subtracting a known or estimated empty weight of the cryogenic fluid supply vessel 204 from the total weight indicated by the sensor 222 to obtain a weight of cryogen present or available in the cryogenic fluid supply vessel 204. Additionally, the determined weight of the cryogenic fluid present in the cryogenic fluid supply vessel 204 can be converted to a present or available volume of cryogenic fluid by a known conversion factor, look up table, or similar approach.

The determined weight and/or volume of the cryogenic fluid present or available in the cryogenic fluid supply vessel 204 can be displayed to the operator, e.g., via the display 108 (FIG. 1) or other visual or audio indicator. Additionally, the determined weight of the cryogen present or available in the cryogenic fluid supply vessel 204 can be converted to an available run time, so that the operator can decide whether the available amount of cryogenic fluid is sufficient for a planned procedure. The available run time can be calculated as a function of the amount of cryogenic fluid available and one or more known, constant flow rates of the probe 106. Additionally or alternatively, the display can provide a binary go/no go indicator based on a time required for a procedure chosen or input by the operator and the determined available amount of cryogenic fluid.

The control system can be configured to provide information to the operator regarding the available amount of cryogenic fluid only when the sensor 222 is able to provide accurate information regarding the overall weight of the cryogenic fluid supply vessel 204. For example, in the configuration shown in FIG. 2B in which the cryogenic fluid supply vessel 204 is sealed against the interface 212, a reaction force between the cryogenic fluid supply vessel 204 and the interface 212 could result in erroneous signals from the sensor 222. Additionally, in the configuration shown in FIG. 2B, pressurized air applied to the cryogenic fluid to force the cryogenic fluid from the cryogenic fluid supply vessel 204 and through the cryogen inlet tube 218 can also alter the force applied to the cryogenic fluid supply vessel 204 and thereby affect the signal from the sensor 222. Accordingly, the control system 224 is configured to provide information from the sensor 222 regarding the available amount of cryogenic fluid on the condition that the cryogenic fluid supply vessel 204 is in a position lowered from the position shown in FIG. 2B, e.g., is not in a fully raised position or is not in a fully engaged position. For example, in some embodiments, the control system 224 is configured to provide information based on the signal from the sensor 222 only on the condition that the cryogenic fluid supply vessel 204 is in the lowered position shown in FIG. 2A. In other embodiments, the control system can be configured to provide the information in any position of the cryogenic fluid supply vessel 204 below the fully raised position of FIG. 2B.

In some embodiments, the cryogen delivery system 200 can be configured to carry out a cryoablation procedure including multiple cooling cycles in which the cryogen is applied to a treatment area via the probe 206, and thawing periods in between the cooling cycles. Such procedures are discussed in more detail in U.S. Patent No. 8,092,448 incorporated by reference herein. In some situations, an operator may wish to know how much cryogen is available after carrying out a portion, e.g., a cooling cycle, of a procedure. Thus, according to some exemplary embodiments of the present disclosure, during a thawing period or other time in which cryogen is not being drawn from the cryogenic fluid supply vessel 204, the cryogenic fluid supply vessel 204 may be lowered from the position in which it is sealed against the interface 212 to an at least partially lowered position, (e.g., to ensure reaction forces of the interface 212 against the mouth 214 of the cryogen supply vessel 204 do not influence the signal from the sensor) and the available amount of cryogen indicated to the operator. At this point, the operator can decide whether the available amount is sufficient to complete any other cooling cycles of the selected procedure and/or the cryogen delivery system can indicate, e.g., with a go/no go indicator whether an available amount of cryogen is sufficient to complete the selected procedure.

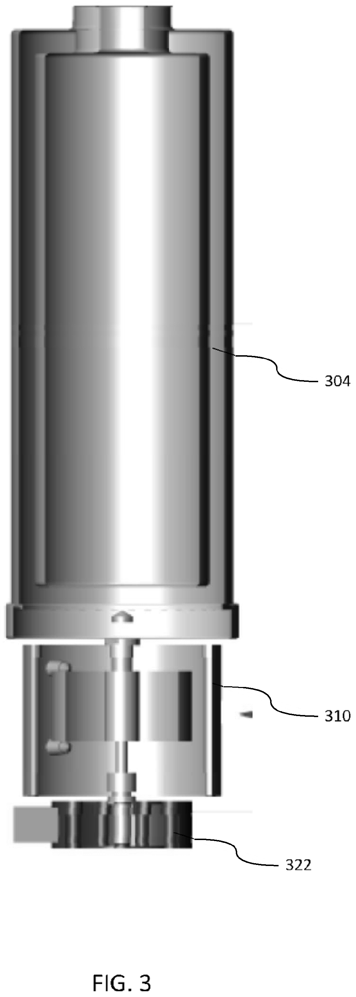

Referring now to FIG. 3, a cross-sectional side view of a moveable support structure, sensor, and cryogenic fluid supply vessel according to an exemplary embodiment of the present disclosure is shown. In this embodiment, a sensor 322 is located below moveable support structure 310. With this arrangement, the weight of the cryogenic fluid supply vessel 304 and the moveable support structure 310 is applied to the sensor 322 and, thus, the sensor 322 can be calibrated using the weight of the moveable support structure 310 and empty weight of the cryogenic fluid supply vessel 304. That is, any weight indicated by the sensor 322 beyond the weight of the moveable support structure 310 and the empty weight of the cryogenic fluid supply vessel 304 represents the weight of the cryogen in the cryogenic fluid supply vessel 304.

The moveable support structure 310 can comprise a hydraulic lift, a pneumatic lift, an electromechanical lift, or other system. In some exemplary embodiments, the moveable support structure 310 is a pneumatic lift operable with the air pump 220 (FIGS. 2A and 2B) and actuatable via a user input device, e.g., a touchscreen display (such as display 108 shown in FIG. 1), an actuatable switch, or other control operable by the operator. Once the moveable support structure 310 raises the cryogenic fluid supply vessel 304 to the fully raised position shown in FIG. 2B, the moveable support structure 310 can be configured to apply a constant force to the cryogenic fluid supply vessel 304 sufficient to maintain a seal between the cryogenic fluid supply vessel 304 and the interface 212 (FIG. 2B) to prevent loss of the cryogen via evaporation.

The sensor 322 can comprise, for example, a strain gauge, a load cell comprising an arrangement of multiple strain gauges, such as in a Wheatstone bridge configuration, a piezoelectric transducer, or any other device configured to generate a signal in response to an applied load. While the sensor 322 is shown in FIG. 3 as supporting the moveable support structure 310, other configurations are within the scope of the present disclosure. For example, and without limitation, the sensor 322 may be positioned between the moveable support structure 310 and the cryogenic fluid supply vessel 304 or may be positioned on a portion of the moveable support structure 310 in any location in which a weight of the cryogenic fluid supply vessel 104 generates a signal from the sensor 322.

In some embodiments, storage areas for cryogenic fluid supply vessels of a cryogen delivery system can also be provided with weight sensors. For example, as shown in FIG. 1, storage area 103 can be provided with sensors similar to sensor 322, and the cryogen delivery system can be configured to provide information to the operator regarding the amount of cryogen present or available in additional cryogenic fluid supply vessels 104B and 104C (FIG. 1). The information regarding the amount of cryogen available in the additional cryogenic fluid supply vessels 104B and 104C can be provided to the operator in a similar manner as described in connection with fluid supply vessel 104A, e.g., may be provided in terms of available weight, available volume, available run time, etc.

In some embodiments, the cryogen delivery system can include an automated cryogenic fluid supply vessel switching system configured to automatically switch between cryogenic fluid supply vessels 104A, 104B, and 104C (FIG. 1) based on a detected amount of cryogen available in each of cryogenic fluid supply vessels 104A, 104B, and 104C. For example, in some example embodiments, the storage area 103 (FIG. 1) can include an automated system configured to automatically reposition each of cryogenic fluid supply vessels 104A, 104B, and 104C in turn on the moveable support structure 310. The automated movement of the system can be driven by a control system of the cryogen delivery system or automated movement may be initiated, via user input, based on detected amounts of cryogen in each of the cryogenic fluid supply vessels 104A, 104B, 104C.

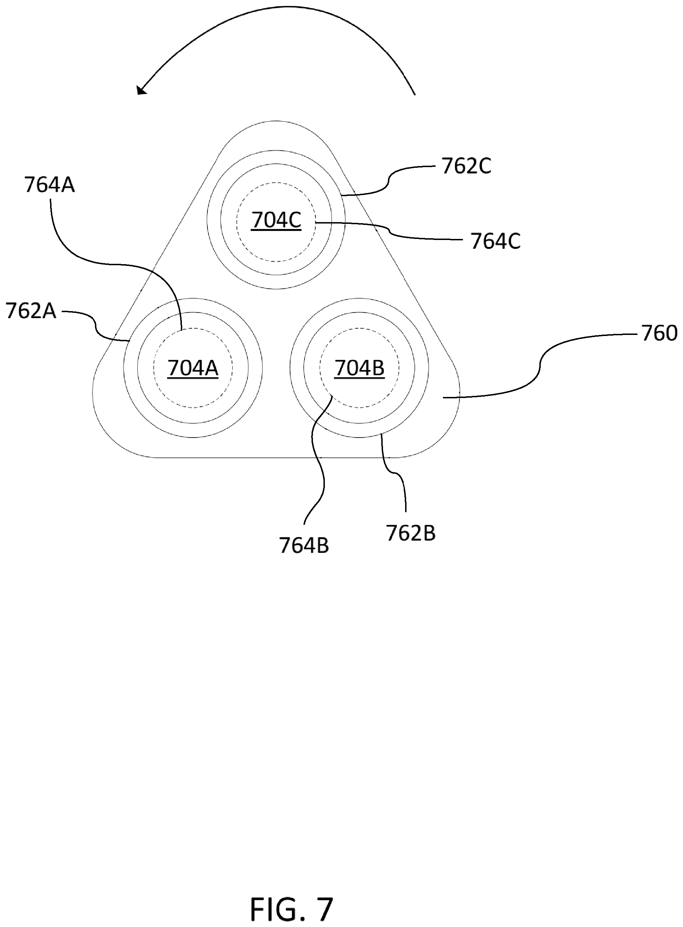

Referring now to FIG. 7, an automated support platform 760 is configured to rotate automatically (e.g., as driven by a motor or other actuator operably coupled with a control system of a cryogen delivery system) based on a detected amount of cryogen in each of fluid supply vessels 704A, 704B, 704C. In this embodiment, each storage location 762A, 762B, 762C on support platform 760 that is configured to receive a cryogenic fluid supply vessel 704A, 704B, 704C, is operably associated with a sensor 764A, 764B, 764C, each of which is operably associated with the control system and configured to provide to the control system information regarding the total weight of each cryogenic fluid supply vessel 704A, 704B, 704C, thereby indicating an available amount of cryogen in each fluid supply vessel 704A, 704B, 704C. The sensors 764A, 764B, and 764C may be located beneath each fluid supply vessel 704A, 704B, 704C, and are indicated by dashed lines in FIG. 7. However, other arrangements are considered within the scope of the present disclosure, and the sensors may be configured differently in different embodiments, such as coupled to a cantilever support or otherwise not located beneath the cryogenic fluid supply vessels. Each sensor 764A, 764B, 764C can be or include a strain gauge, piezoelectric transducer, or other device as discussed herein. In the embodiment of FIG. 7, a fluid supply vessel 704A is in position to be raised by a moveable support structure (such as moveable support structure 310 shown in FIG. 3) to engage with an interface (such as interface 212 shown in FIGS. 2A and 2B) to deliver cryogen for use in a cryoablation procedure. Upon depletion of the cryogen available in fluid supply vessel 704A, fluid supply vessel 704A can be lowered from the interface 212. The automated support platform 760 can be rotated (for example, either automatically, as part of a disengagement process, or in response to input by an operator of the cryogenic system), bringing another fluid supply vessel (e.g., fluid supply vessel 704C) into position to be raised by the moveable support structure. In some embodiments, the system can include a single moveable support structure 310, and the automated system can reposition each of the cryogenic fluid vessels on the moveable support structure 310. Alternatively, each of the storage locations 762A, 762B, 762C can include a separate moveable support structure configured to position each of the cryogenic fluid supply vessels 704A, 704B, 704C relative to the interface 212 (FIG. 2A and 2B).

While the embodiment of FIG. 7 includes a rotating automated support platform 760, other devices for automatically switching between cryogenic fluid supply vessels are within the scope of the disclosure. For example, some embodiments may include one or more of a conveyor, a moveable carriage, an automated gripping member such as a robotic arm, or other device. Further, while the embodiment of FIG. 7 includes three cryogenic fluid supply vessels 704A, 704B, 704C, other embodiments may include more than three cryogenic fluid supply vessels or fewer than three cryogenic fluid supply vessels associated with an automated switching system. Further, the automated switching system may perform switching between cryogenic fluid supply vessels based on a detected amount of available cryogen in each cryogenic fluid supply vessel and a known amount required for a selected cryoablation procedure or a portion of a selected cryoablation procedure. Thus, rather than providing a go/no go indicator as discussed further herein when an available amount of cryogen is insufficient to complete a procedure or a portion of a procedure, the control system may command the automated support platform or other automated system to switch fluid supply vessels to a fluid supply vessel with sufficient cryogen to complete the procedure or portion of the procedure.

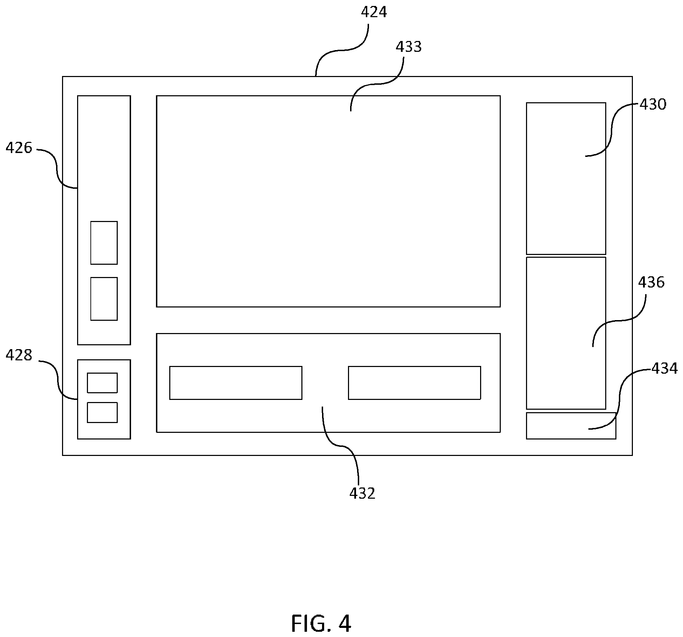

FIG. 4 is a diagram view of a display 424 of a cryogen delivery system according to an exemplary embodiment of the present disclosure. In the embodiment of FIG. 4, the display 424 comprises a touchscreen with various controls related to the operation of the cryoablation system. For example, the display can include, among other things, vessel controls 426 for raising and lowering the cryogenic fluid supply vessel, probe controls 428 for locking out use of the probe 106 (FIG. 1) to prevent inadvertent actuation of the cryogen delivery system, a status area 430 for communicating various messages to the operator, a mode selection area 432, a shutdown button 434, and a general display field 433 to display other information used for operation of the cryoablation system.

In some embodiments, the display 424 can include a cryogen status indicator area 436 that is configured to display, based on the signal from the sensor 322 (FIG. 3), the amount of cryogen available in the cryogenic fluid supply vessel 304 (FIG. 3). As discussed above, the cryogen status indicator area 436 can provide the information as an available weight, an available volume, a maximum procedure run time, and/or a binary go/no-go indicator that indicates whether the amount of cryogen present/available is sufficient to complete a procedure chosen by the operator. The cryogen status indicator area 436 can be configured to display the information regarding the available amount of cryogen numerically, e.g., by actual weights or volumes, by percentages (based on weight/volume of full fluid supply vessel), or can be configured to display the information graphically, such as with a bar graph, gauge, or other graphical representation. Further, the display can be configured such that the operator can choose a desired type of information display. That is, in some embodiments, the operator can choose whether to view the information as an available weight, available volume, maximum run time, etc. The cryogen status indicator area 436 can be configured to display the information regarding the available amount of cryogen via text, colors, icons, warning lights (flashing or steady state) on the display or on a portion of the console (e.g., console 100 shown in FIG. 1), or any combination thereof. The visual indicators may be combined with audio indicators such as speech reciting displayed text indicators, beeping, or other audible indicators.

While the embodiment of FIG. 4 is configured to display the information regarding available cryogen via the display 424, other embodiments can include other indicators, such as visual indicators not associated with the display 424, audio indicators, or other combinations of indicators. Further, in other exemplary embodiments, the various controls and information areas can be relocated, reconfigured, or combined in different ways without departing from the scope of the present disclosure.

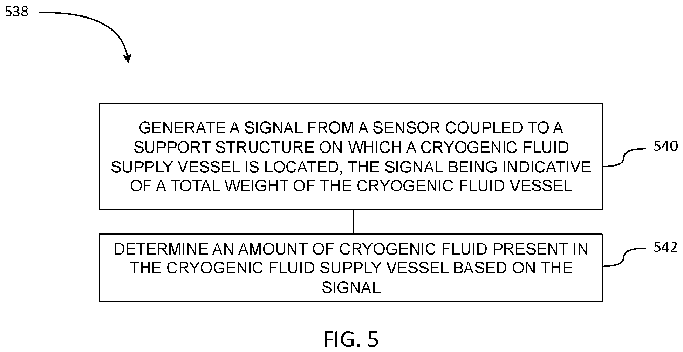

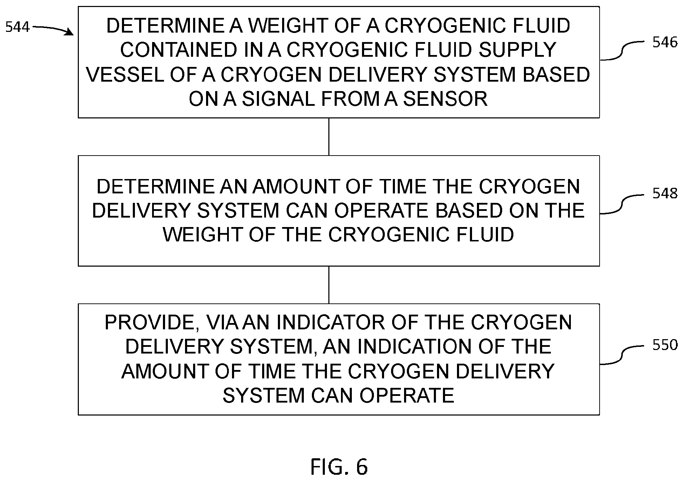

Referring now to FIG. 5, an example workflow 538 for a cryogen delivery procedure is shown. At 540, a signal from a sensor coupled to a support structure is on which a cryogenic fluid supply vessel is located is generated, the signal being indicative of a total weight of the cryogenic fluid supply vessel. At 542, an amount of cryogenic fluid present in the cryogenic fluid supply vessel is determined based on the signal. Referring now to FIG. 6, another example workflow 544 for a cryogen delivery procedure is shown. At 546, a weight of a cryogenic fluid contained in a cryogenic fluid supply vessel of a cryogen delivery system is determined based on a signal from a sensor. At 548, an amount of time the cryogen delivery system can operate is determined based on the weight of the cryogenic fluid. At 550, an indication of the amount of time the cryogen delivery system can operate is provided via an indicator of the cryogen delivery system. According to some embodiments, the control system may provide an indication to the operator indicating whether the available amount of cryogen is sufficient to carry out the second cooling procedure based on the available amount of cryogen, a known flow rate associated with the procedure (e.g., a known flow rate through probe 106 (FIG. 1)), and a known timeframe of the second cooling procedure, e.g., based on a procedure chosen by the operator and input via the a mode selection area 432 of the display 424 (FIG. 4). As discussed above, this indication may be in the form of a go/no go indicator presented to the operator, e.g., via the display 424, such as at the cryogen status indicator area 436 (FIG. 4).

The run times of various procedures can be stored by the control system, e.g., in electronic memory, and may be based on known run times chosen by an operator. These run times can be based on, for example, the size and shape or other characteristics of a mass or lesion. In some exemplary embodiments, the procedure times can be as disclosed in U.S. Patent No. 8,092,448, incorporated above. For example, depending on the size of the mass to be treated, the procedure may include two cooling (e.g., freezing) cycles occurring before and after a passive thawing period. As non-limiting examples, each cooling cycle can be in a range of from about 1 minute to about 8 minutes, and the thawing period can be in a range of from about 2 minutes to about 10 minutes. In some embodiments, the two cooling cycles are equal in length, and the thawing period is longer than each of the cooling cycles. In other embodiments, the thawing period can be equal or less than the time of the cooling cycles, the cooling cycle times can differ from one another, the procedure may include more thawing periods and/or more cooling cycles, and other changes may be made based on the desired result of the procedure.

Devices and systems according to the present disclosure can be used, for example, for cryoablation procedures used for treatment of both benign and cancerous tumors. In one example according to the disclosure of U.S. Patent No. 8,092,448, incorporated above, a fibroadenoma with a major axis of 3.1 to 3.5 cm diameter is treated by two cycles of freezing, each consisting of 6 minutes of freezing, with 10 minutes of passive thawing between the freezing cycles. As an additional example, a fibroadenoma of 3.6 to 4.0 cm diameter is treated by two cycles of freezing, each consisting of 8 minutes of freezing, with 10 minutes of passive thawing between the freezing cycles. Further examples for fibroadenomas of different sizes and shapes are provided in U.S. Patent No. 8,092,448.

Systems according to the present disclosure can increase the efficiency and efficacy of cryoablation procedures by providing useful information to the operator of the cryogen delivery system regarding the amount of cryogen present in a cryogenic fluid supply vessel.

### Examples

Illustrative examples of the systems methods described herein are provided below. An embodiment of the system and method described herein may include any one or more, and any combination of, the clauses described below:
Clause 1. A cryogen delivery system, comprising:
   a probe configured to deliver a cryogenic fluid to a treatment location;
   a cryogen inlet configured to interface with a cryogenic fluid supply vessel to transfer cryogenic fluid from the cryogenic fluid supply vessel to the probe;
   a support structure configured to position the cryogenic fluid supply vessel relative to the cryogen inlet; and
   a sensor coupled to the support structure and configured to generate a signal indicative of a weight of a cryogenic fluid contained in the cryogenic fluid supply vessel.
Clause 2. The system of clause 1, wherein the sensor comprises a strain gauge sensor.
Clause 3. The system of any of clauses 1-2, wherein the support structure is a moveable support structure configured to move the cryogenic fluid supply vessel from a first position to a second position, the second position being a position in which the cryogenic fluid supply vessel is interfaced with the cryogen inlet.
Clause 4. The system of clause 3, wherein the moveable support structure comprises a pneumatic lift or an electromechanical lift.
Clause 5. The system of any of clauses 1-4, wherein the cryogen delivery system further comprises a visual interface configured to display information based on the signal indicative of the weight of the cryogenic fluid contained in the cryogenic fluid supply vessel.
Clause 6. The system of clause 5, wherein the visual interface is configured to display a volume of the cryogenic fluid present in the cryogenic fluid supply vessel based on the signal indicative of the weight of the cryogenic fluid contained in the cryogenic fluid supply vessel.
Clause 7. The system of any of clauses 5-6, wherein the visual interface is configured to display an amount of run time for operation of the cryogen delivery system based on the signal indicative of the weight of the cryogenic fluid contained in the cryogenic fluid supply vessel.
Clause 8. The system of any of clauses 5-7, wherein the visual interface is configured to display a percentage representative of an amount of cryogenic fluid present in the cryogenic fluid supply vessel, based on the signal indicative of the weight of the cryogenic fluid, as compared to an amount of cryogenic fluid present in a full cryogenic fluid supply vessel.
Clause 9. The system of any of clauses 1-8, wherein the cryogen delivery system is configured to perform cryoablation.
Clause 10. The system of clause 9, wherein the cryogen delivery system is configured to perform a plurality of cooling cycles in which the probe delivers the cryogenic fluid to the treatment location.
Clause 11. The system of clause 10, wherein the cryogen delivery system is configured to compare an amount of cryogenic fluid present in the cryogenic fluid supply vessel with an amount of cryogenic fluid available to perform a procedure.
Clause 12. The system of clause 11, wherein the cryogen delivery system is configured to indicate whether the amount of cryogenic fluid present in the cryogenic fluid supply vessel is sufficient to complete a selected procedure.
Clause 13. The system of any of clauses 1-12, wherein:
   the cryogenic fluid supply vessel comprises a plurality of cryogenic fluid supply vessels; and
   the cryogen delivery system comprises an automated fluid supply vessel switching system configured to move a first cryogenic fluid supply vessel of the plurality of cryogenic fluid supply vessels from the support structure and place a second cryogenic fluid supply vessel of the plurality of cryogenic fluid supply vessels on the support structure based at least in part on a weight of a cryogenic fluid contained in the first cryogenic fluid supply vessel.
Clause 14. A method for determining an amount of cryogenic fluid in a cryogenic fluid supply vessel, the method comprising:
   generating a signal from a sensor coupled to a support structure on which a cryogenic fluid supply vessel is located, the signal being indicative of a total weight of the cryogenic fluid supply vessel; and
   determining an amount of cryogenic fluid present in the cryogenic fluid supply vessel based on the signal.
Clause 15. The method of clause 14, wherein generating a signal from a sensor coupled to the support structure comprises generating a signal from a strain gauge sensor coupled to the support structure.
Clause 16. The method of any of clauses 14-15, wherein determining an amount of a cryogenic fluid in the cryogenic fluid supply vessel comprises subtracting an estimated weight of an empty cryogenic fluid supply vessel from the total weight of the cryogenic fluid supply vessel.
Clause 17. The method of any of clauses 14-16, further comprising providing an indication regarding the amount of cryogenic fluid present in the cryogenic fluid supply vessel.
Clause 18. The method of clause 17, wherein providing an indication regarding the amount of cryogenic fluid present in the cryogenic fluid supply vessel comprises one or more of a visible indication and an audible indication.
Clause 19. The method of clause 18, wherein providing an indication regarding the amount of cryogenic fluid present in the cryogenic fluid supply vessel includes displaying the indication on a display associated with the cryogen delivery system.
Clause 20. The method of any of clauses 17-19, wherein providing the indication regarding the amount of cryogenic fluid present in the cryogenic fluid supply vessel comprises providing an indication of the amount of cryogenic fluid present in the cryogenic fluid supply vessel by weight.
Clause 21. The method of any of clauses 17-20, wherein providing the indication regarding the amount of cryogenic fluid present in the cryogenic fluid supply vessel comprises providing an indication of the amount of cryogenic fluid present in the cryogenic fluid supply vessel by volume.
Clause 22. The method of any of clauses 17-20, wherein providing the indication regarding the amount of cryogenic fluid present in the cryogenic fluid supply vessel comprises providing an indication the amount of cryogenic fluid present is sufficient to complete a freezing cycle of the cryogen delivery system, providing an indication the amount of cryogenic fluid present is insufficient to complete a freezing cycle of the cryogen delivery system; or providing an indication that the amount of cryogenic fluid present is sufficient to complete two freezing cycles of the cryogen delivery system.
Clause 23. A method for determining sufficiency of an amount of cryogen for performing a procedure with a cryogen delivery system, the method comprising:
   determining a weight of a cryogenic fluid contained in a cryogenic fluid supply vessel of the cryogen delivery system based on a signal from a sensor;
   determining an amount of time the cryogen delivery system can operate based on the weight of the cryogenic fluid; and
   providing, via an indicator of the cryogen delivery system, an indication of the amount of time the cryogen delivery system can operate.
Clause 24. The method of clause 23, wherein determining a weight of a cryogenic fluid contained in a cryogenic fluid supply vessel comprises:
   determining a total weight of the cryogenic fluid supply vessel and the cryogenic fluid; and
   subtracting the weight of the cryogenic fluid supply vessel from the total weight.
Clause 25. The method of any of clauses 23-24, wherein providing an indication of the amount of time the cryogen delivery system can operate comprises displaying a visual indicator on a display operatively associated with the cryogen delivery system.
Clause 26. The method of any of clauses 23-25, further comprising receiving an input at a user input device of the cryogen delivery system indicative of a selected cryoablation procedure;
   determining a runtime of the selected cryoablation procedure; and
   determining whether the amount of time the cryogen delivery system can operate is less than, equal to, or greater than the runtime of the selected cryoablation procedure.
Clause 27. The method of clause 26, wherein, on the condition the amount of time the cryogen delivery system can operate is less than the runtime of the selected cryoablation procedure, providing an indication of the amount of time the cryogen delivery system can operate comprises providing an indication that the amount of cryogen is insufficient to perform the selected cryoablation procedure.
Clause 28. The method of clause 27, wherein, on the condition the amount of time the cryogen delivery system can operate is greater than the runtime of the selected cryoablation procedure, providing an indication of the amount of time the cryogen delivery system can operate comprises providing an indication that the amount of cryogen is sufficient to perform the selected cryoablation procedure.
Clause 29. The method of clause 28, wherein the selected cryoablation procedure includes a first runtime corresponding to a first freezing cycle and a second runtime corresponding to a second freezing cycle and, wherein, determining whether the amount of time the cryogen delivery system can operate is less than, equal to, or greater than the runtime of the selected cryoablation procedure comprises determining whether the amount of time the cryogen delivery system can operate is less than, equal to, or greater than the first runtime and/or the first and second runtimes.

This disclosure described some examples of the present technology with reference to the accompanying drawings, in which some of the possible examples were shown. Other aspects can, however, be embodied in many different forms and should not be construed as limited to the examples set forth herein. Rather, these examples were provided so that this disclosure was thorough and complete and fully conveyed the scope of the possible examples to those skilled in the art.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

This description and the accompanying drawings that illustrate exemplary embodiments should not be taken as limiting. Various mechanical, compositional, structural, electrical, and operational changes may be made without departing from the scope of this description and the claims, including equivalents. In some instances, well-known structures and techniques have not been shown or described in detail so as not to obscure the disclosure. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be included in the second embodiment.

It is noted that, as used herein, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

Further, this description's terminology is not intended to limit the disclosure. For example, spatially relative terms-such as "beneath," "below," "lower," "above," "upper," "forward," "front," "behind," and the like-may be used to describe one element's or feature's relationship to another element or feature as illustrated in the orientation of the figures. These spatially relative terms are intended to encompass different positions and orientations of a device in use or operation in addition to the position and orientation shown in the figures. For example, if a device in the figures is inverted, elements described as "below" or "beneath" other elements or features would then be "above" or "over" the other elements or features. Thus, the exemplary term "below" can encompass both positions and orientations of above and below. A device may be otherwise oriented (rotated 90 degrees or at other orientations) and the spatially relative descriptors used herein interpreted accordingly.

Further modifications and alternative embodiments will be apparent to those of ordinary skill in the art in view of the disclosure herein. For example, the systems may include additional components that were omitted from the diagrams and description for clarity of operation. Accordingly, this description is to be construed as illustrative only and is for the purpose of teaching those skilled in the art the general manner of carrying out the systems and methods of the present disclosure. It is to be understood that the various embodiments shown and described herein are to be taken as exemplary. Elements and materials, and arrangements of those elements and materials, may be substituted for those illustrated and described herein, parts and processes may be reversed, and certain features of the present teachings may be utilized independently, all as would be apparent to one skilled in the art after having the benefit of the description herein. Changes may be made in the elements described herein without departing from the scope of the present disclosure.

It is to be understood that the particular examples and embodiments set forth herein are non-limiting, and modifications to structure, dimensions, materials, and methodologies may be made without departing from the scope of the present disclosure. Other embodiments in accordance with the present disclosure will be apparent to those skilled in the art from consideration of the specification and practice of the invention disclosed herein. It is intended that the specification and examples be considered as exemplary only, with being entitled to their full breadth of scope, including equivalents.

## Claims

1. A cryogen delivery system (200), comprising:
a probe (206) configured to deliver a cryogenic fluid to a treatment location;
a cryogen inlet (212) configured to interface with a cryogenic fluid supply vessel (204) to transfer cryogenic fluid from the cryogenic fluid supply vessel (204) to the probe (206);
a support structure (210) configured to position the cryogenic fluid supply vessel (204) relative to the cryogen inlet (212);
a sensor (222) coupled to the support structure (210) and configured to generate a signal indicative of a weight of a cryogenic fluid contained in the cryogenic fluid supply vessel (204); and
a control system (224) operably coupled to the sensor (222), wherein the control system (224) is configured to sense an amount of cryogenic fluid in the cryogenic fluid supply vessel (204) only when the cryogenic fluid supply vessel (204) is not interfaced with the cryogen inlet (212).

2. The system (200) of claim 1, wherein the sensor (222) comprises a strain gauge sensor.

3. The system (200) of claim 1, wherein the support structure (210) is a moveable support structure configured to move the cryogenic fluid supply vessel (204) from a first position to a second position, the second position being a position in which the cryogenic fluid supply vessel (204) is interfaced with the cryogen inlet (212).

4. The system (200) of claim 3, wherein the moveable support structure (210) is configured to raise and lower the cryogenic fluid supply vessel (204) relative to the cryogen inlet (212).

5. The system (200) of any one of claims 3-4, wherein the moveable support structure (210) is configured to create or break a seal between the cryogenic fluid supply vessel (204) and the cryogen inlet (212).

6. The system (200) of claim 5 when dependent on claim 4, wherein the control system (224) is configured to provide information based on the signal from the sensor (222) only on the condition that the cryogenic fluid supply vessel (204) is in a lowered position in which the cryogenic fluid supply vessel (204) can be placed on or removed from the moveable support structure (210).

7. The system (200) of any one of claims 3-6, wherein the moveable support structure (210) comprises a pneumatic lift or an electromechanical lift.

8. The system (200) of claim 5, further comprising a source of pressurised gas (220) configured to pressurise the interior of the cryogenic fluid supply vessel (204) when the cryogenic fluid supply vessel (204) is sealed with the cryogen inlet (212), to force the cryogenic fluid from the cryogenic fluid supply vessel (204) through the cryogen inlet (212) to the probe (206).

9. The system (200) of any one of claims 4-8, wherein the cryogen delivery system (200) is configured to carry out a cryoablation procedure including multiple cooling cycles in which the cryogenic fluid is applied to the treatment location via the probe (206) and thawing periods in between the cooling cycles, and wherein, during a thawing period, the moveable support structure (210) is configured to lower the cryogenic fluid supply vessel (204) from a position in which the cryogenic fluid supply vessel (204) is sealed against the cryogen inlet (212) to an at least partially lowered position, and the control system (224) is configured to indicate the amount of cryogenic fluid to an operator.

10. The system (200) of any one of claims 1-9, wherein the control system (224) is configured to sense the amount of cryogenic fluid by subtracting a known or estimated empty weight of the cryogenic fluid supply vessel (204) from a total weight of the cryogenic fluid supply vessel (204) indicated by the sensor (222).

11. The system (200) of any one of claims 1-10, wherein the cryogen delivery system (200) further comprises a visual interface configured to display information based on the signal indicative of the weight of the cryogenic fluid contained in the cryogenic fluid supply vessel (204), the information preferably comprising one or more of a volume of the cryogenic fluid present in the cryogenic fluid supply vessel (204), an amount of run time for operation of the cryogen delivery system (200), and a percentage representative of the amount of cryogenic fluid in the cryogenic fluid supply vessel (204) as compared to an amount of cryogenic fluid present in a full cryogenic fluid supply vessel.

12. The system (200) of any one of claims 1-11, wherein the cryogen delivery system (200) is configured to perform cryoablation and is preferably configured to perform a plurality of cooling cycles in which the probe (206) delivers the cryogenic fluid to the treatment location.

13. The system (200) of claim 12, wherein the cryogen delivery system (200) is configured to perform a plurality of cooling cycles in which the probe (206) delivers the cryogenic fluid to the treatment location and to compare the amount of cryogenic fluid in the cryogenic fluid supply vessel (204) with an amount of cryogenic fluid required to perform a procedure, and is preferably configured to indicate whether the amount of cryogenic fluid in the cryogenic fluid supply vessel (204) is sufficient to complete a selected procedure.
